# EUROPEAN PATENT SPECIFICATION

(11) **EP 1 257 277 B1**
(45) Date of publication and mention of the grant of the patent: **15.06.2005**
(21) Application number: 01910421.5
(22) Date of filing: 23.02.2001
(51) Int. Cl.: A61K 31/535, A61K 31/135, A61P 13/10

(54) **NEW DRUG COMBINATIONS COMPRISING A NOREPINEPHRINE REUPTAKE INHIBITOR AND AN ANTIMUSCARINIC AGENT**
NEUE WIRKSTOFFKOMBINATION, ENTHALTEND EINEN INHIBITOR DER EPINEPHRINAUFNAHME SOWIE EINEN ANTIMUSKARINEN WIRKSTOFF
NOUVELLES COMBINAISONS MEDICAMENTEUSES

(30) Priority: 24.02.2000 US 184790 P
(43) Date of publication of application: 20.11.2002
(73) Proprietor: Pharmacia & Upjohn Company LLC, Kalamazoo, MI 49001 (US)
(72) Inventor: ROGOSKY, Karen, Pittstown, New Jersey 08867 (US); JORN, Deborah, Warren, NJ 07059 (US)
(74) Representative: Duncan, Garreth Andrew
(86) International application number: PCT/US2001/003698
(87) International publication number: WO 2001/062236

(56) References cited:
- EP-A- 0 368 682
- WO-A-97/09036
- PHARMACIA & UPJOHN: "pharmacia & upjohn" FORMULARY'S RESOURCE GUIDE, vol. 34, no. 10 suppl., October 1999 (1999-10), pages 74-78, XP001013175

## Description

### Field of the Invention

This invention relates to new drug combinations intended to provide rapid onset of relief from several nervous system disorders, and incontinence.

### Background of the Invention

The introduction of tricyclic antidepressants in the early 1960s provided a major advance in the treatment of neuropsychiatric disorders. Reactive and endogenous depressions, diagnoses formerly carrying grave prognostic implications, have become, with the introduction of the tricyclics, manageable disorders with a much smaller toll on patients and society as a whole.

The early tricyclic compounds were reuptake inhibitors of all the catecholamines released in the synaptic deft, thus resulting in prolongation and enhancement of the dopamine (DA), noradrenaline (NA) and serotonin (5-hydroxytryptamine = 5-HT) action. Lack of selectivity also causes undesired side-effects, particularly on neurotransmission mediated by acetylcholine (especially the muscarinic component) and histamine.

These unwanted pharmacodynamic activities, cognitive impairment, sedation, urinary and gastrointestinal tract disturbances, and increased intraocular pressure, were limiting factors in the clinical use of these compounds and often required discontinuation of treatment. Of utmost concern were also the cardiac toxic effects and the proconvulsant activity of this group of drugs.

More recently, selective reuptake inhibitors for serotonin (SSRI) have been introduced with definite advantages in regard to fewer side effects without loss of efficacy. Fluoxetine is an example of such an inhibitor that has had a great amount of commercial success.

Another class of compounds that has been proposed for use in the treatment of depression is selective norepinephrine reuptake inhibitors. Lower-than-normal levels of norepinephrine are associated with a variety of symptoms including lack of energy, motivation, and interest in life. Thus, a normal level of norepinephrine is essential to maintaining drive and capacity for reward. These neurotransmitters travel from the terminal of a neuron across a small gap (i.e., the synaptic cleft) and bind to receptor molecules on the surface of a second neuron. This binding elicits intracellular changes that initiate or activate a response or change in the postsynaptic neuron. Inactivation occurs primarily by transport (i.e., reuptake) of the neurotransmitter back into the presynaptic neuron. Abnormality in noradrenergic transmission results in various types of depression, mental, behavioral, and neurological disorders attributed to a variety of symptoms including a lack of energy, motivation, and interest in life. *See generally,* R.J. Baldessarini, "Drugs and the Treatment of Psychiatric Disorders: Depression and Mania" in *Goodman and Gilman's The Pharmacological Basis of Therapeutics*, McGraw-Hill, NY, NY, pp. 432-439 (1996).

Examples of norepinephrine reuptake inhibitors (both selective and not selective) include, the following: tandamine (CAS 42408-80-0; US 3904617; US 4118394), pirandamine (CAS 42408-79-7; US 3995052), ciclazindol (CAS 37751-39-6; US 3891644; US 3957819; US 3976645), fluparoxan (US 4880801), lortalamine (CAS 70384-91-7; US 4201783), talsupram (CAS 21489-20-3), talopram (CAS 7182-51-6), prindamine, nomifensine (US 3577424), viloxazine (US 3712890), tomoxetine (US 4314081), duloxetine (US 5023269), venlafaxine (US 4535186), milnacipran (US 4478836) and reboxetine (US 4229449). Tomoxetine, (R)-(-)-N-methyl-3-(2-methylphenyoxy)-3-phenylpropylamine is disclosed in U.S. Patent No. 4,314,081. Reboxetine, 2-[alpha-(2-ethoxy)phenoxybenzyl]morpholine is disclosed in U.S. Patent No. 4,229,449. Reboxetine includes both the racemate, as well as the (-)(R,R) and (+)(S,S) enantiomers. This product is also identified by the following trademarks: VESTRA, PROLIFT, NOREBOX and EORONAX. Duloxetine, N-methyl-3-(1-naphthalenyloxy)-3-(2-thienyl) propanamine is disclosed in U.S. Patent No. 4,956,388. It is usually administered as the hydrochloride salt and as the (+) enantiomer. Venlafaxine is identified as Compound A of U.S. Patent No. 4,761,501. Milnacipran, N,N-diethyl-2-aminomethyl-1-phenylcyclopropanecarboxamide is disclosed in U.S. Patent No. 4,478,836.

Antimuscarinic agents can be used to treat urinary incontinence. Examples of antimuscarinic agents include, the following: tolterodine, propiverine, oxybutynin, trospium, darifenacin, temiverine, ipratropium.

Tolterodine, Phenol, 2-[3-[bis(1-methylethyl)amino]-1-phenylpropyl]-4-methyl-, (R)-, an antimuscarinic with a high degree of bladder selectivity, has been developed and launched by Pharmacia & Upjohn under the DETROL® trademark for the treatment of incontinence associated with an overactive bladder. This product is disclosed in U.S. Patent No. 5,382,600. The active metabolites of tolterodine are

Propiverine is 1-methyl-4-piperidyl .alpha.,.alpha.-diphenyl-.alpha.-(n-propoxy)acetate and is disclosed in East German Patent No. 106643 and in CAS 82-155841s (1975). Oxybutynin is 4-(Diethylamino)-2-butynylalphaphenylcyclohexaneglycolate and is disclosed in UK Patent No. 940540. Trospium is 3alpha-Hydroxyspiro[1alphaH,5alphaH-nortropane-8,1'-pyrrolidinium]chloride benzilate and is disclosed in U.S. Patent No. 3480623. Darifenacin is 3-Pyrrolidineacetamide, 1-[2-(2,3-dihydro-5-benzofuranyl)ethyl]-alpha,alpha-diphenyl-, and is disclosed in U.S. Patent No. 5,096,890. Temiverine is Benzeneacetic acid, .alpha.-cyclohexyl-.alpha.-hydroxy-, 4-(diethylamino)-1,1-dimethyl-2-butynyl ester and is disclosed in US-A-5096890. Temiverine is Benzeneacetic acid, .alpha.-cyclohexyl-.alpha.-hydroxy-, 4-(diethylamino)-1,1-dimethyl-2-butynyl ester and is disdosed in US-A-5036098. Ipratropium is 8-isopropytnoratropine methobromide and is disclosed in US-A-3505337.

### Summary of the Invention

A first aspect of the present invention is a composition comprising:
(a) one or more norepinephrine reuptake inhibitors or pharmaceutically effective salts thereof; and
(b) one or more antimuscarinic agents or pharmaceutically effective salts thereof.

A second aspect of the invention is a composition comprising components (a) and (b), as a combined preparation for separate, sequential or simultaneous use, for the treatment of incontinence or a disease or disorder of the central nervous system wherein said disease or disorder is selected from obesity, depression, schizophrenia, stress-related diseases (e.g. general anxiety disorder), panic disorder, phobias, obsessive compulsive disorder, post-traumatic stress syndrome, immune system depression, incontinence, a stress-induced problem with the urinary, gastrointestinal or cardiovascular system, neurodegenerative disorders, autism, chemotherapy-induced vomiting, hypertension, migraine headaches, cluster headaches, sexual dysfunction in a mammal, addictive disorder and withdrawal syndrome, adjustment disorders, age-associated learning and mental disorders, anorexia nervosa, apathy, attention-deficit disorders due to general medical conditions, attention-deficit hyperactivity disorder, bipolar disorder, bulimia nervosa, chronic fatigue syndrome, conduct disorder, cyclothymic disorder, dysthymic disorder, fibromyalgia and other somatoform disorders, generalised anxiety disorder, inhalation disorders, intoxication disorders, movement disorders, oppositional defiant disorder, pain disorders, peripheral neuropathy, post-traumatic stress disorder, premenstrual dysphoric disorder, psychotic disorders, seasonal affective disorder, sleep disorders, specific developmental disorders, and selective serotonin reuptake inhibition (SSRI) "poop out" syndrome.

Components (a) and (b) may be used for the manufacture of a medicament for treating incontinence or a disease or disorder of the central nervous system, as defined above.

Preferably, component (a) comprises reboxetine in either its enantiomeric or racemic form, and component (b) comprises tolterodine, including its active metabolites.

### Description of the Preferred Embodiment

In describing the preferred embodiment, certain terminology will be utilized for the sake of clarity. Such terminology is intended to encompass the recited embodiment, as well as all technical equivalents which operate in a similar manner for a similar purpose to achieve a similar result. To the extent that any pharmaceutically active compound is disclosed or ctaimed, it is expressly intended to include all active metabolites produced *in vivo*, and is expressly intended to include all enantiomers, isomers or tautomers where the compound is capable of existing in an enantiomeric, isomeric or tautomeric form. The first component is a norepinephrine reuptake inhibitor, with selective norepinephrine reuptake inhibitors being particularly preferred. This list of compounds includes, the following: tandamine, pirandamine, ciclazindol, fluparoxan, lortalamine, talsupram, talopram, prindamine, nomifensine, viloxazine, tomoxetine, duloxetine, venlafaxine, milnacipran and reboxetine, with reboxetine being particularly preferred.

Examples of pharmaceutically effective salts for the selective norepinephrine reuptake inhibitor include, salts prepared from pharmaceutically acceptable acids or bases, including organic and inorganic acids and bases. When the preferred compound of use is basic (for example reboxetine), salts may be prepared from pharmaceutically acceptable acids. Suitable pharmaceutically acceptable acids include acetic, benzenesulfonic (besylate), benzoic, p-bromophenylsulfonic, camphorsulfonic, carbonic, citric, ethanesulfonic, fumaric, gluconic, glutamic, hydrobromic, hydrochloric, hydroiodic, isethionic, lactic, maleic, malic, mandelic, methanesulfonic (mesylate), mucic, nitric, oxalic, pamoic, pantothenic, phosphoric, succinic, sulfuric, tartaric, p-toluenesulfonic, and the like. Examples of such pharmaceutically acceptable salts include, , acetate, benzoate, hydroxybutyrate, bisulfate, bisulfite, bromide, butyne-1,4-dioate, carpoate, chloride, chlorobenzoate, citrate, dihydrogenphosphate, dinitrobenzoate, fumarate, glycollate, heptanoate, hexyne-1,6-dioate, hydroxybenzoate, iodide, lactate, maleate, malonate, mandelate, metaphosphate, methanesulfonate, methoxybenzoate, methylbenzoate, monohydrogenphosphate, naphthalene-1-sulfonate, naphthalene-2-sulfonate, oxalate, phenylbutyrate, phenylproionate, phosphate, phthalate, phylacetate, propanesulfonate, propiolate, propionate, pyrophosphate, pyrosulfate, sebacate, suberate, succinate, sulfate, sulfite, sulfonate, tartrate, xylenesulfonate, and the like.

In particularly preferred embodiments the selective norepinephrine reuptake inhibitor is reboxetine, 2-[α-((2-ethoxyphenoxy)benzyl]-morpholine, and its pharmaceutically acceptable salts, in either its enantiomeric (particularly the (S,S) enantiomer) or racemic form. Synthesis of racemic reboxetine is described in greater detail in U.S. Patent No. 4,229,449. Individual stereoisomers of reboxetine can be obtained by resolution of the racemic mixture of enantiomers using conventional methods generally known by those skilled in the art. Such methods include, resolution by simple crystallization and chromatographic techniques, for example, as set forth in GB 2,167,407. Other methods of preparation are described in US 5,068,433 and US 5,391,735. Reboxetine can be a free base form, or it can be in salt form, preferably the methanesulfonate salt (also called reboxetine mesylate).

The selection of the dosage of the first component is that which can provide relief to the patient. As is well known, the dosage of this component depends on several factors such as the potency of the selected specific compound, the mode of administration, the age and weight of the patient, the severity of the condition to be treated, and the like. This is considered to be within the skill of the artisan and one can review the existing literature on the components to determine optimal dosing.

Desirably, when reboxetine is selected as the active agent, the daily dose contains from about 0.1mg. to about 10 mg. More preferably, each dose of the component contains about 0.5 to about 8 mg of the active ingredient, and even more preferably, each dose contains from about 0.5 to about 5 mg of the active ingredient. This dosage form permits the full daily dosage to be administered in one or two oral doses. This will allow for final formulations containing 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9. 2.0, 2.1, 2.2, 2.3, 2.4, or 2.5 mg of active. More than once daily or twice daily administrations (e.g., 3, 4, 5 or 6 administrations per day) are also expressly contemplated herein.

The average daily adult dosage of the other norepinephrine reuptake inhibitors is as follows. The dosages expressly include all numerical values, whole or fractional, within the stated range. Pediatric dosages may be less.

| | |
|---|---|
| Component | Average Daily Dosage (mg/day/patient) |
| Tandamine | 7.5 to 3750 |
| Pirandamine | 7.5 to 3750 |
| Ciclazindol | 5 to 500 |
| Fluparoxan | .75 to 750 |
| Lortalamine | 1 to 200 |
| Talsupram | 1 to 3750 |
| Talopram | 1 to 3750 |
| Prindamine | 1 to 3750 |
| Nomifensine | 1 to 80 |
| Viloxazine | 1 to 3750 |
| Tomoxetine | 1 to 200 |
| Duloxetine | 5 to 500 |
| Venlafaxine | 2 to 200 |
| Milnacipran | 7.5 to 75 |

The second component comprises one or more antimuscarinic agents. Examples of such agents include tolterodine, propiverine, oxybutynin, trospium, darifenacin, temiverine and ipratropium. Particularly preferred is tolterodine.

The chemical name of tolterodine is Phenol, 2-[3-[bis(1-methylethyl)amino]-1-phenylpropyl]4-methyl-, (R)-, including its pharmaceutically active salts, such as those described above with respect to the first active component and its active metabolites that are produced *in vivo.* Synthesis of tolterodine is disclosed in U.S. Patent No. 5,382,600. This compound is particularly useful as an anticholingeric agent, and more specifically for the treatment of incontinence.

As is well known, the dosage and administrative regimen (i.e., one, two, three or more administrations per day) of the second component depends on the factors referred to in connection with the dosage selection of the first component. To the extent necessary for completion, the synthesis of the components and dosages described in the patents or CAS documents referenced in the Technology Description portion of this document are expressly incorporated by reference. The average adult daily dosage of the second component is from about 0.05 mg to about 5 mg per kilogram of body weight, administered in one or more doses, e.g. containing from about 0.05 to about 250 mg each. The dosages expressly include all numerical values, whole or fractional, within the stated range. Pediatric dosages may be less.

Compositions of the present invention can conveniently be administered in a pharmaceutical composition containing the active components in combination with a suitable excipient. Such pharmaceutical compositions can be prepared by methods and contain excipients which are well known in the art. A generally recognized compendium of such methods and ingredients is Remington's Pharmaceutical Sciences by E.W. Martin (Mark Publ. Co., 15th Ed., 1975). The compositions of the present invention can be administered parenterally (for example, by intravenous, intraperitoneal, subcutaneous or intramuscular injection), topically, orally, intranasally, intravaginally, or rectally, with oral administration being particularly preferred.

For oral therapeutic administration, the inventive composition may be combined with one or more excipients and used in the form of ingestible tablets, buccal tablets, troches, capsules, elixirs, suspensions, syrups, wafers, chewing gums, foods and the like. Such compositions and preparations should contain at least 0.1% of active compound. The percentage of the compositions and preparations may, of course, be varied and may conveniently be between about 0.1 to about 100% of the weight of a given unit dosage form. The amount of active compound in such therapeutically useful compositions is such that an effective dosage level will be obtained.

The tablets, troches, pills, capsules, and the like may also contain the following: binders such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, fructose, lactose or aspartame or a flavoring agent such as peppermint, oil of wintergreen, or cherry flavoring. . When the unit dosage form is a capsule, it may contain, in addition to materials of the above type, a liquid carrier, such as a vegetable oil or a polyethylene glycol. Various other materials may be present as coatings or to otherwise modify the physical form of the solid unit dosage form. For instance, tablets, pills, or capsules may be coated with gelatin, wax, shellac or sugar and the like. A syrup or elixir may contain the active compound, sucrose or fructose as a sweetening agent, methyl and propylparabens as preservatives, a dye and flavoring such as cherry or orange flavor. Of course, any material used in preparing any unit dosage form should be pharmaceutically acceptable and substantially non-toxic in the amounts employed. In addition, the active components may be incorporated into sustained-release preparations and devices including, but not limited to, those relying on osmotic pressures to obtain a desired release profile. Once daily formulations for each of the active components are specifically included.

The inventive composition, containing the two active components, may be administered in the same physical form or concomitantly according to the above-described dosages and in the above-described delivery vehicles. The dosages for each active component can be measured separately and can be given as a single combined dose or given separately. They may be given at the same or at different times as long as both actives are in the patient at one time over a 24-hour period. Concomitant or concurrent administration means the patient takes one drug within about 5 minutes of taking the other drug. Because the goal is to provide rapid symptomatic relief to the patient, in most cases when treatment is started the two drugs would be administered to the patient close in time and typically concomitantly; thereafter, the timing of each drug's administration may not be as important.

The novel composition is used to treat incontinence (of any type) or any of the given diseases or disorders of the central nervous system. Such diseases and disorders are defined in The Diagnostic and Statistical Manual of Mental Disorders-IV (DSM-IV) (American Psychiatric Association (1995)).

Treatment is accomplished by delivering a therapeutically effective amount of the composition to a mammal. In most cases, this will be a human being, but treatment of food animals (e.g., livestock and poultry) and companion animals (e.g., dogs, cats and horses) is expressly covered herein.

In particular, the novel composition is to be used in the treatment of incontinence (i.e., stress incontinence, genuine stress incontinence, and mixed incontinence). Stress urinary incontinence is a symptom describing involuntary loss of urine on carrying out any activity that raises intra-abdominal pressure, such as coughing or sneezing. Stress incontinence is also a clinical sign, that is the observation by a care giver of a jet of urine escaping from the urethral meatus (opening) when the patient coughs or strains. Genuine Stress Incontinence (urge incontinence) is the pathological diagnosis of an incompetent urethral sphincter as diagnosed by Urodynamic testing. Mixed incontinence is stress incontinence in combination with urge incontinence. The latter is a part of the symptom complex of the Overactive Bladder. Retention may be due to outflow obstruction (e.g., high urethral pressure), poor detrusor (bladder muscle) contractility or lack of coordination between detrusor contraction and urethral relaxation. The inventive drug combination can be used in connection with stress incontinence, urge incontinence or mixed incontinence.

The novel composition is expected to provide rapid relief to those suffering from the above diseases or disorders with a minimal amount of deleterious side effects.

The invention is described in greater detail by the following example.

### Example 1

A pharmaceutical composition is prepared by combining reboxetine in either its racemic or (S,S) enantiomeric form with tolterodine in a pharmaceutically acceptable carrier. The composition contains respective amounts of reboxetine and tolterodine to deliver on a daily basis between about 0.1 mg to about 10 mg reboxetine and between about 0.05 mg to about 4 mg of tolterodine per kilogram of patient body weight (for example, 3 mg to 240 mg tolterodine for a person weighing 60 kg). The composition is administered to a patient for the treatment of incontinence, and particularly stress incontinence, urge incontinence or mixed incontinence.

### Example 2

A first pharmaceutical composition is prepared by combining reboxetine in either its racemic or +(S,S) enantiomeric form in a pharmaceutically acceptable carrier such that it can deliver between about 0.1 mg to about 10 mg reboxetine on a daily basis. A second pharmaceutical composition is prepared by combining tolterodine in a pharmaceutically acceptable carrier such that it can deliver between about 0.05 mg to about 4 mg of tolterodine per kilogram of patient body weight on a daily basis. The first composition is administered to a patient suffering from one or more forms of incontinence once, twice, three times, four times or six times daily such that the daily dosage is between about 0.1 to about 10 mg. The second composition is administered to the same patient at the same time as the administration of the first composition or any time within 24 hours of the administration of the first composition once, twice, three times, four times or six times daily such that the daily dosage is between about 0.05 mg to about 4 mg of tolterodine per kilogram of patient body weight. Alternatively, the second composition could first be administered, followed by the administration of the first composition as disclosed at the same time, or within 24 hours thereof.

## Claims

1. A composition comprising:
(a) one or more norepinephrine reuptake inhibitors or pharmaceutically effective salts thereof; and
(b) one or more antimuscarinic agents or pharmaceutically effective salts thereof.

2. A composition according to claim 1, wherein component (a) is selected from tandamine, pirandamine, ciclazindol, fluparoxan, lortalamine, talsupram, talopram, prindamine, nomifensine, viloxazine, tomoxetine, duloxetine, venlafaxine, milnacipran, reboxetine and mixtures thereof.

3. A composition according to claim 1 or claim 2, wherein component (b) is selected from tolterodine, propivame, oxybutynin, trospium, darifenacin, temiverine, ipratropium and mixtures thereof.

4. A composition according to claim 1, wherein component (a) is reboxetine in either its racemic or +(S,S) enantiomeric form.

5. A composition according to claim 4, wherein component (b) is tolterodine or an active metabolite thereof.

6. A composition according to claim 4, wherein component (b) is darifenacin.

7. A composition according to claim 4, wherein component (b) is trospium.

8. A composition according to claim 4, wherein component (b) is ipratropium.

9. A composition according to claim 1, wherein component (a) is duloxetine.

10. A composition according to claim 9, wherein component (b) is selected from tolterodine, darifenacin, trospium, ipratropium and mixtures thereof.

11. A composition comprising component (a) and component (b) as defined in any of claims 1 to 10, as a combined preparation for separate, sequential or simultaneous use, for the treatment of incontinence or a disease or disorder of the central nervous system, wherein said disease or disorder is selected from obesity, depression, schizophrenia, stress-related diseases (e.g. general anxiety disorder), panic disorder, phobias, obsessive compulsive disorder, post-traumatic stress syndrome, immune system depression, incontinence, a stress-induced problem with the urinary, gastrointestinal or cardiovascular system, neurodegenerative disorders, autism, chemotherapy-induced vomiting, hypertension, migraine headaches, cluster headaches, sexual dysfunction in a mammal, addictive disorder and withdrawal syndrome, adjustment disorders, age-associated learning and mental disorders, anorexia nervosa, apathy, attention-deficit disorders due to general medical conditions, attention-deficit hyperactivity disorder, bipolar disorder, bulimia nervosa, chronic fatigue syndrome, conduct disorder, cyclothymic disorder, dysthymic disorder, fibromyalgia and other somatoform disorders, generalised anxiety disorder, inhalation disorders, intoxication disorders, movement disorders, oppositional defiant disorder, pain disorders, peripheral neuropathy, post-traumatic stress disorder, premenstrual dysphoric disorder, psychotic disorders, seasonal affective disorder, sleep disorders, specific developmental disorders, and selective serotonin reuptake inhibition (SSRI) "poop out" syndrome.

12. A composition according to any of claims 1 to 11, wherein component (a) and component (b) are provided in the same delivery vehicle.

13. A composition according to any of claims 1 to 11, wherein component (a) and component (b) are maintained in different delivery vehicles.

14. Use of component (a) and component (b) as defined in any of claims 1 to 10, for the manufacture of a medicament for treating incontinence or a disease or disorder of the central nervous system as defined in claim 11.

15. Use according to claim 14, for treating incontinence.

16. Use according to claim 15, wherein the incontinence is stress incontinence, genuine stress incontinence or mixed incontinence.

17. Use according to any of claims 15 to 16, wherein the medicament is adapted to be administered rectally, topically, orally, sublingually, intranasally, transdermally or parenterally.

## Patentansprüche

1. Zusammensetzung, welche umfasst:
(a) einen oder mehrere Norepinephrin-Wiederaufnahmehemmer oder pharmazeutisch wirksame Salze davon; und
(b) einen oder mehrere Antimuscarin-Wirkstoffe oder pharmazeutisch wirksame Salze davon.

2. Zusammensetzung nach Anspruch 1, wobei die Komponente (a) aus Tandamin, Pirandamin, Ciclazindol, Fluparoxan, Lortalamin, Talsupram, Talopram, Prindamin, Nomifensin, Viloxazin, Tomoxetin, Duloxetin, Venlafaxin, Milnacipran, Reboxetin und Gemischen davon ausgewählt ist.

3. Zusammensetzung nach Anspruch 1 oder 2, wobei die Komponente (b) aus Tolterodin, Propiverin, Oxybutynin, Trospium, Darifenacin, Temiverin, Ipratropium und Gemischen davon ausgewählt ist.

4. Zusammensetzung nach Anspruch 1, wobei die Komponente (a) Reboxetin entweder in seiner racemischen oder +(S,S)-enantiomeren Form vorliegt.

5. Zusammensetzung nach Anspruch 4, wobei die Komponente (b) Tolterodin oder ein aktiver Metabolit davon ist.

6. Zusammensetzung nach Anspruch 4, wobei die Komponente (b) Darifenacin ist.

7. Zusammensetzung nach Anspruch 4, wobei die Komponente (b) Trospium ist.

8. Zusammensetzung nach Anspruch 4, wobei die Komponente (b) Ipratropium ist.

9. Zusammensetzung nach Anspruch 1, wobei die Komponente (a) Duloxetin ist.

10. Zusammensetzung nach Anspruch 9, wobei die Komponente (b) aus Tolterodin, Darifenacin, Trospium, Ipratropium und Gemischen davon ausgewählt ist.

11. Zusammensetzung, umfassend Komponente (a) und Komponente (b) wie in irgend einem der Ansprüche 1 bis 10 definiert, als kombinierte Zubereitung für die separate, sequenzielle oder simultane Verwendung zur Behandlung von Inkontinenz oder von einer Erkrankung oder Störung des Zentralnervensystems, wobei besagte Erkrankung oder Störung aus Adipositas, Depression, Schizophrenie, stress-abhängigen Erkrankungen (z.B. generalisierte Angststörung), Panikstörungen, Phobien, obsessiver Zwangsstörung, posttraumatischem Belastungssyndrom, Beeinträchtigung des Immunsystems, Inkontinenz, stress-induzierten Problemen mit dem Harn-, Gastrointestinal- oder kardiovaskulären System, neurodegenerativen Störungen, Autismus, durch Chemotherapie induziertes Erbrechen, Hypertonie, Migränekopfschmerzen, Clusterkopfschmerzen, sexueller Dysfunktion in einem Säuger, Suchtstörungen und Entzugssyndrom, Anpassungsstörungen, alters-abhängigen Lern- und Mentalstörungen, Anorexia nervosa, Apathie, Aufmerksamkeitsdefizienz-Störungen infolge allgemeiner medizinischer Zustände,
Aufmerksamkeitsdefizienz-Hyperaktivitäts-Störung, bipolarer Störung, Bulimia nervosa, Chronic-Fatigue-Syndrom, Verhaltensstörung, Zyklothymie-Störung, Dysthymie-Störung, Fibromyalgie und anderen somatoformen Störungen, generalisierter Angststörung, Inhalationsstörungen, Intoxikationsstörungen, Bewegungsstörungen, oppositionelle Verhaltensstörung, Schmerzstörungen, peripherer Neuropathie, posttraumatischer Belastungsstörung, prämenstruellem dysphorischem Syndrom, psychotischen Störungen, saisonalen affektiven Störungen, Schlafstörungen, spezifischen Entwicklungsstörungen und dem "Poop-Out"-Syndrom selektiver Serotoninwiederaufnahmehemmer ausgewählt ist.

12. Zusammensetzung nach irgend einem der Ansprüche 1 bis 11, wobei die Komponente (a) und die Komponente (b) in demselben Verabreichungsvehikel bereitgestellt sind.

13. Zusammensetzung nach irgend einem der Ansprüche 1 bis 11, wobei die Komponente (a) und die Komponente (b) in unterschiedlichen Vehikeln enthalten sind.

14. Verwendung der Komponente (a) und der Komponente (b) wie in irgend einem der Ansprüche 1 bis 10 definiert, zur Herstellung eines Medikamentes zur Behandlung von Inkontinenz oder von einer Erkrankung oder Störung des Zentralnervensystems wie in Anspruch 11 definiert.

15. Verwendung nach Anspruch 14 zur Behandlung von Inkontinenz.

16. Verwendung nach Anspruch 15, wobei die Inkontinenz eine Stress-Inkontinenz, eine eigentliche Stress-Inkontinenz oder eine gemischte Inkontinenz ist.

17. Verwendung nach irgend einem der Ansprüche 15 bis 16, wobei das Medikament für die rektale, topische, orale, sublinguale, intranasale, transdermale oder parenterale Verabreichung angepasst ist.

## Revendications

1. Composition comprenant :
(a) un ou plusieurs inhibiteurs de la recapture de la norépinéphrine ou sels pharmaceutiquement acceptables de tels inhibiteurs ; et
(b) une ou plusieurs agents antimuscariniques ou sels pharmaceutiquement acceptables de tels agents.

2. Composition selon la revendication 1, dans laquelle le composant (a) est sélectionné parmi la tandamine, la pirandamine, le ciclazindol, le fluparoxan, la lortalamine, le talsupram, le talopram, la prindamine, la nomifensine, la viloxazine, la tomoxétine, la duloxétine, la venlafaxine, le milnacipran, la réboxétine et leurs mélanges.

3. Composition selon la revendication 1 ou la revendication 2, dans laquelle le composant (b) est sélectionné parmi la toltérodine, la propivérine, l'oxybutynine, le trospium, la darifénacine, la témivérine, l'ipratropium et leurs mélanges.

4. Composition selon la revendication 1, dans laquelle le composant (a) est la réboxétine soit sous sa forme racémique, soit sous sa forme énantiomère +(S,S).

5. Composition selon la revendication 4, dans laquelle le composant (b) est la toltérodine ou un métabolite actif de ce composé.

6. Composition selon la revendication 4, dans laquelle le composant (b) est la darifénacine.

7. Composition selon la revendication 4, dans laquelle le composant (b) est le trospium.

8. Composition selon la revendication 4, dans laquelle le composant (b) est l'ipratropium.

9. Composition selon la revendication 1, dans laquelle le composant (a) est la duloxétine.

10. Composition selon la revendication 9, dans laquelle le composant (b) est sélectionné parmi la toltérodine, la darifénacine, le trospium, l'ipratropium et leurs mélanges.

11. Composition comprenant le composant (a) et le composant (b) tels que définis dans l'une quelconque des revendications 1 à 10, sous la forme d'une préparation combinée, en vue d'une utilisation séparée, successive ou simultanée, pour le traitement de l'incontinence ou d'une maladie ou d'un trouble du système nerveux central, ladite maladie ou trouble étant sélectionné parmi l'obésité, la dépression, la schizophrénie, les maladies liées au stress (par exemple les troubles de l'anxiété généralisée, les troubles paniques, les phobies, les troubles obsessionnels compulsifs, le syndrome de stress post-traumatique, la dépression du système immunitaire, l'incontinence, un problème du système urinaire, gastro-intestinal ou cardio-vasculaire induit par le stress, les troubles neurodégénératifs, l'autisme, les vomissements induits par une chimiothérapie, l'hypertension, les migraines, l'algie vasculaire de la face, la dysfonction sexuelle chez les mammifères, les troubles de la dépendance et du sevrage, les troubles d'adaptation, les troubles mentaux et de l'apprentissage liés à l'âge, l'anorexie nerveuse, l'apathie, les troubles du déficit d'attention dus à un état médical général, les troubles d'hyperactivité avec déficit d'attention, les troubles bipolaires, la boulimie nerveuse, le syndrome de fatigue chronique, les troubles du comportement, les troubles cyclothymiques, les troubles dysthymiques, la fibromyalgie, et d'autres troubles somatoformes, les troubles de l'anxiété généralisée, les troubles liés à l'inhalation de vapeurs, les troubles d'intoxication, la dyskinésie, les troubles d'opposition-défiance, les troubles douloureux, la neuropathie périphérique, les troubles de stress post-traumatique, les troubles dysphoriques prémenstruels, les troubles psychotiques, les troubles affectifs saisonniers, les troubles du sommeil, les troubles spécifiques de développement et le syndrome "poop out" d'inhibition de la recapture sélective de la sérotonine.

12. Composition selon l'une quelconque des revendications 1 à 11, dans laquelle le composant (a) et le composant (b) sont fournis dans le même véhicule de distribution.

13. Composition selon l'une quelconque des revendications 1 à 11, dans laquelle le composant (a) et le composant (b) sont maintenus dans des véhicules de distribution différents.

14. Utilisation du composant (a) et du composant (b) tels que définis dans l'une quelconque des revendications 1 à 10, pour la fabrication d'un médicament pour le traitement de l'incontinence ou d'une maladie ou d'un trouble du système nerveux central, tels que définis dans la revendication 11.

15. Utilisation selon la revendication 14, pour le traitement de l'incontinence.

16. Utilisation selon la revendication 15, dans laquelle l'incontinence est une incontinence à l'effort, une incontinence à l'effort vraie ou une incontinence mixte.

17. Utilisation selon l'une quelconque des revendications 15 à 16, dans laquelle le médicament est adapté à une administration par voie rectale, topique, orale, sublinguale, intranasale, transdermique ou parentérale.
